# EUROPÄISCHE PATENTSCHRIFT

(11) **EP 3 346 969 B1**
(45) Veröffentlichungstag und Bekanntmachung des Hinweises auf die Patenterteilung: **14.07.2021**
(21) Anmeldenummer: 16757625.5
(22) Anmeldetag: 23.08.2016
(51) Int. Cl.: A61K 6/30, A61K 6/887

(54) **RADIKALISCH POLYMERISIERBARES DENTALMATERIAL**
RADICALLY POLYMERIZABLE DENTAL MATERIAL
MATÉRIAU DENTAIRE POLYMÉRISABLE PAR VOIE RADICALAIRE

(30) Priorität: 11.09.2015 DE 102015217418
(43) Veröffentlichungstag der Anmeldung: 18.07.2018
(73) Patentinhaber: Mühlbauer Technology GmbH, 22547 Hamburg (DE)
(72) Erfinder: BECKER, Olav-Sven, 20253 Hamburg (DE); NEFFGEN, Stephan, 25421 Pinneberg (DE)
(74) Vertreter: Glawe, Delfs, Moll
(86) Internationale Anmeldenummer: PCT/EP2016/069880
(87) Internationale Veröffentlichungsnummer: WO 2017/042025

(56) Entgegenhaltungen:
- EP-A1- 1 169 996
- US-A- 4 499 251
- US-B2- 8 404 144

## Beschreibung

Die Erfindung betrifft ein radikalisch polymerisierbares Dental-material.

Radikalisch polymerisierbare Dentalmaterialien, insbesondere Dentaladhäsive, sind im Stand der Technik bekannt. Ebenfalls bekannt sind sogenannte selbstätzende Dentalmaterialien, die auch ohne Vorbehandlung (Ätzen mittels Säure und/oder Primen) auf Zahnhartsubstanz haften.

US 4,499,251 offenbart N-substituierte (Meth)acrylamido-bisphosphonsäuren für adhäsive dentale Zusammensetzungen. Die Haftwerte der Beispielzusammensetzungen auf Schmelz sind gering.

Catel et al. [Polym Int 2013; 62: 1717-28] beschreibt die Verwendung von 10-(N-methylacrylamido)decylbisphosphonsäure für eine Primerzusammensetzung (Tabelle 4) zur Verwendung mit einem nachfolgend anzuwendenden Adhäsiv. Die Zweischrittigkeit ist nachteilig.

US 8, 404, 144 offenbart eine Zusammensetzung, die eine oder mehrere polymerisierbare Bisphosphonsäuren in Kombination mit einer oder mehreren zusätzlichen polymerisierbaren Komponenten enthält. Die erfindungsgemäße Zusammensetzung ist als selbstätzender Primer und selbstätzendes Adhäsiv beschrieben.

Der vorliegenden Erfindung liegt die Aufgabe zu Grunde, Dentalmaterialien der eingangs genannten Art zu schaffen, die in einem Schritt angewendet werden können und ohne Vorbehandlung der Zahnhartsubstanz mittels Primer und/oder Ätzmittel eine gute Haftung auf Zahnhartsubstanz, insbesondere Schmelz, bewirken. Die Verwendung der in der vorliegenden Erfindung beschriebenen Materialien kann überdies vorteilhaft sein bei der Haftvermittlung zu Oberflächen von Zahnrestaurationen aus anorganischen Materialien wie Oxidkeramiken, Silikatkeramiken oder Metallen, insbesondere bei der Haftvermittlung zu Oxidkeramiken.

Gegenstand der Erfindung ist ein Dentalmaterial, das enthält:
a. radikalisch polymerisierbare Monomere gemäß Formel I: 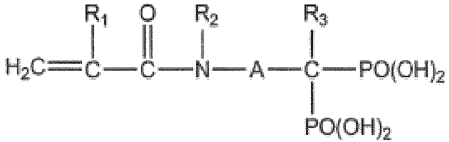 mit:
   R1 = Methyl oder H
   R2 = Alkyl, Aryl, oder Arylalkyl
   R3 = H, Alkyl oder OR4
   R4 = H oder Alkyl
   A = C6-C18-Alkylen oder C8-C18-Alkylarylen;
b. radikalisch polymerisierbare Monomere enthaltend mindestens eine Acrylat- und/oder Methacrylatgruppe;
c. wenigstens einen Initiator für die radikalische Polymerisation.

Zunächst seien einige im Rahmen der Erfindung verwendete Begriffe erläutert.

Dentalmaterialien im Sinne der Erfindung sind Materialien, die im Zuge der Zahnbehandlung entweder als Restaurationsmaterialien oder im Zuge der Vorbereitung einer Restauration oder der Verbindung anderer Restaurationsmaterialien mit Zahnhartsubstanz (beispielsweise mittels Adhäsiven) Verwendung finden können. Ein erfindungsgemäßes Dentalmaterial kann beispielsweise ausgebildet sein als Bonding, Adhäsiv, Fissurenversiegler, Zement, Befestigungszement, Füllungsmaterial oder Komposit. Die auf das Dental-material gerichteten Patentansprüche beanspruchen das Material im noch nicht polymerisierten bzw. nicht ausgehärteten Zustand.

Das erfindungsgemäße Dentalmaterial kann in einem Schritt angewendet werden. Anwendung in einem Schritt bedeutet, dass das erfindungsgemäße Material ohne vorherige oder nachherige Anwendung eines anderen Materials erfolgreich, d.h. unter Ausbildung einer guten Haftung der zu befestigenden Systeme, angewendet werden kann. Das erfindungsgemäße Material kann selbst das zu befestigende System sein. Sofern es sich bei dem erfindungsgemäßen Material um ein Adhäsiv handelt, kann dieses bspw. ohne die vorherige Verwendung eines Primers mit gutem Erfolg angewendet werden. Andererseits ist es für den Fall einer Verwendung des erfindungsgemäßen als Adhäsiv auch nicht erforderlich, nach seiner Anwendung eine zweite Komponente (häufig als Bond bezeichnet) zur Haftvermittlung mit einem in der Folge anzuwendenden Restaurativ oder Zement, welche häufig auf Methacrylatbasis formuliert sind, aufzubringen.

In einer anderen Verwendung ist das erfindungsgemäße Material ein selbstadhäsives Befestigungsmaterial (Zement) zur Befestigung prothetischer Restaurationen wie Kronen und Brücken. Der erfindungsgemäße Zement kann ohne vor- oder nachherige Anwendung eines zweiten Materials erfolgreich eingesetzt werden.

In einer weiteren Verwendung ist das erfindungsgemäße Material ein Restaurativ oder ein Fissurenversiegler. Auch diese Anwendungsformen lassen sich ohne weitere Schritte auf den entsprechenden Substraten mit guter Adhäsion befestigen.

Die Verwendung der in der vorliegenden Erfindung beschriebenen Materialien kann überdies vorteilhaft sein bei der Haftvermittlung zu Oberflächen von Zahnrestaurationen aus anorganischen Materialien wie Oxidkeramiken, Silikatkeramiken oder Metallen, insbesondere bei der Haftvermittlung zu Oxidkeramiken.

Bei dem erfindungsgemäßen Material kann es sich auch um ein einoder ein mehrkomponentiges Material handeln.

Im Falle eines einkomponentigen Materials kann dieses als ein Komponentensystem gelagert und angewendet werden, es ist nicht erforderlich, kurz vor oder während der Verwendung zwei oder mehr Komponenten zu mischen. Dass Einkomponentensystem wird direkt auf das Substrat aufgebracht und dort ausgehärtet. Dies kann bevorzugt durch die Bestrahlung mit Licht geschehen.

Im Falle eines mehrkomponentigen Materials werden die Komponenten vor der Anwendung vermischt und die Mischung auf dem Substrat appliziert. Eine mehrkomponentige Formulierung kann bspw. sinnvoll sein, um eine vollständige Aushärtung in Situationen zu erreichen, in denen eine Erhärtung mit Licht nicht möglich ist. Mehrkomponentige Formulierungen können sowohl in einer Verwendung als Adhäsiv als auch in einer Verwendung als Befestigungsmaterial (Zement) oder auch als Restaurativ sinnvoll sein. Mehrkomponentige Materialien sind bevorzugt zweikomponentige Materialien.

In einer Ausprägung sind die erfindungsgemäßen Materialien bevorzugt als zwei- oder mehrkomponentige Materialien ausgeführt, wenn sie durch die Mischung der Komponenten zur Aushärtung gebracht werden sollen. In einem solchen Fall sind die die Er-härtung durch Reaktion miteinander auslösenden Substanzen in unterschiedlichen Komponenten enthalten, um eine vorzeitige und unbeabsichtigte Erhärtung des Materials zu vermeiden.

Mehrkomponentige Materialien können auch bevorzugt sein, um negative Effekte auf die Lagerbarkeit des Materials zu vermeiden.

Bspw. kann es chemische Unverträglichkeiten zwischen der stark sauren Stoffgruppe a. und weiteren Bestandteilen des Materials geben. Es kann bspw. bevorzugt sein, solche Bestandteile, die insbesondere unter Säureeinfluss hydrolyseempfindlich sind aber aus anderen Gründen in Mischung mit Bestandteilen der Stoffgruppe a. vorliegen, von stark wasserhaltigen Bestandteilen zu trennen. Solche Stoffe können bspw. aus den Stoffgruppen b. oder c. stammen. Eine Komponente enthält in bevorzugten Formulierungen dann die Säure sowie weitere damit verträgliche Bestandteile, während eine andere Komponente das Wasser enthält.

Ebenso können bestimmte Bestandteile an sich auch ohne Einfluss von Wasser säureempfindlich sein, sei es dass sie mit der Säure unter Bildung nachteiliger Produkte reagieren (bspw. Salzbildung mit Aminen), sei es dass sie in Gegenwart von Säuren instabil werden und sich zersetzen. Entsprechende säureinduzierte Zersetzungsreaktionen sind dem Fachmann bekannt und seien hier nur beispielhaft genannt: Möglich sind bspw. Disproportionierungsreaktionen von Komponenten aus dem Initiatorbereich (bspw. Sulfinaten) oder beschleunigte Zerfallsreaktionen von Peroxiden unter Säureeinfluss. In bevorzugten Ausführungsformen sind diese, säureempfindlichen Bestandteile dann in den Komponenten enthalten, die keine Säuren und speziell keine Bestandteile der Stoffgruppe a. enthalten.

Nachteilig für die Lagerbarkeit der erfindungsgemäßen Materialien kann es auch sein, wenn Metallverbindungen insbs. Schwermetallverbindungen in Komponenten enthalten sind, die überdies Reduktions- oder Oxidationsmitteln enthalten. Auch solche Reaktionen sind dem Fachmann prinzipiell bekannt. Bespielhaft genannt sei die katalytische Zersetzung von Peroxiden durch Schwermetallverbindungen, die auch zur Selbsterhärtung der jeweiligen Komponenten führen kann, oder die Reduktion von Schwermetallverbindungen zu elementaren Metallen oder unwirksameren Verbindungen niedrigerer Oxidationsstufen durch starke Reduktionsmittel wie Sulfinatsalzen. Überdies sein genannt die bekannte Autooxidationsreaktion von CH-aciden Stoffen in Gegenwart von Cu²⁺. In mehrkomponentigen Materialien sind daher Schwermetallverbindungen bevorzugt in Komponenten enthalten, die keine Peroxide, keine CH-aciden Verbindungen oder auch keine starken Reduktionsmittel enthalten, die mit den Schwermetallverbindungen reagieren können.

Ebenfalls nachteilig für die Lagerbarkeit der erfindungsgemäßen Materialien können Reaktionen starker Nucleophile, bspw. aus der Stoffgruppe c. stammend, mit den elektronenarmen Doppelbindungen der Bestandteile aus den Stoffgruppen a. und b. sein. Exemplarisch genannt sei die Reaktion von Sulfinaten mit (Meth)acrylaten oder anderen elektrophilen ungesättigten Gruppen, die zur chemischen Veränderung der Formulierung führen. Es ist daher bevorzugt, dass starke Nucleophile nicht zusammen mit den Bestandteilen aus den Stoffgruppen a. und b. in einer Komponente enthalten sind. In einer weiteren bevorzugten Ausführungsform, in der ein starkes Nucleophil in einer Komponente zusammen mit Bestandteilen aus den Stoffgruppen a. oder b. enthalten sein kann, ist das starke Nucleophil nicht löslich in der flüssigen Mischung, in denen Bestandteile aus den Stoffgruppen a. oder b. enthalten sind.

Die Erfindung hat erkannt, dass die Verwendung von tertiären, N-substituierten (Meth)acrylamido-Bisphosphonsäuren in Kombination mit einer Kettenlänge des Spacermoleküls zwischen N-substituierter (Meth)Acrylamidogruppe und Bisphosphonsäuregruppe (A in Formel I) von wenigstens C6 überraschenderweise zu einem Dentalmaterial führt, das problemlos gelagert und in einem Schritt angewendet werden kann sowie ohne Vorbehandlung der Zahnhartsubstanz eine Haftfestigkeit insbesondere auf Schmelz erzielt, die im Stand der Technik nicht oder allenfalls mit kompliziert anzuwendenden zwei- oder mehrschrittigen Materialien erreichbar ist.

Das erfindungsgemäße Dentalmaterial ist selbstätzend. Dies bedeutet, dass die Klebefläche der Zahnhartsubstanz (des Dentins) nicht in einem separaten Schritt mittels Säure geätzt zu werden braucht, um gute Haftwerte zu erzielen.

Das erfindungsgemäße Dentalmaterial enthält radikalisch polymerisierbare Monomere, die mindestens eine Acrylat- und/oder Methacrylatgruppe und/oder mindestens zwei Acrylat- und/oder Methacrylatgruppe aufweisen. Bevorzugt enthält das erfindungsgemäße Dentalmaterial radikalisch polymerisierbare Monomere, die eine Acrylat- und/oder Methacrylatgruppe aufweisen, und radikalisch polymerisierbare Monomere, die mindestens zwei Acrylat- und/oder Methacrylatgruppe aufweisen. Bevorzugt liegen diese radikalisch polymerisierbaren Monomere im Verhältnis 1:1 bis 0(Mono(Meth)Acrylat):100(höherfunktionelles (Meth)Acrylat) vor. Bevorzugt enthält das Material für den Fall, dass das Verhältnis (Mono (Meth)Acrylat) : höherfunktionelles (Meth)Acrylat > 0 ist, aus der Gruppe der Monomere mit einer (Meth)Acrylatgruppe mindestens ein (Meth)Acrylat, das eine substantielle Wasserlöslichkeit besitzt und als Phasenvermittler zwischen stark wasserhaltigen Bereichen der Substrate mit schlecht wasserlöslichen Anteilen der Stoffgruppen a. und b. fungieren können. In einer weiteren Ausführungsform enthält das Material eine substantiell wasserlösliche Substanz aus der Gruppe der höherfunktionellen Methacrylate. Substantiell wasserlösliche Substanzen haben bevorzugt eine Löslichkeit in Wasser bei 23°C von mehr als 20 g/L. Als weitere Bestandteile kann das Dentalmaterial zusätzliche radikalisch polymerisierbare Monomere, Oligomere und Präpoly-mere, die mit (Meth)acrylamiden copolymerisieren können, enthalten.

Geeignete Monomere b) mit einer Acrylat- oder Methacrylatgruppe sind beispielsweise Hydroxyethyl(Meth)Acrylat, Hydroxypropyl(Meth)Acrylat, Hydroxybutyl(Meth)Acrylat, Glycerinmono(Meth)Acrylat, Methyl(Meth)Acrylat, Ethyl(Meth)Acrylat, Propyl(Meth)Acrylat, Butyl(Meth)Acrylat, Hexyl(Meth)Acrylat, Octyl(Meth)Acrylat, Lauryl(Meth)Acrylat, Decyl(Meth)Acrylat, Tridecyl(Meth)Acrylat, 2-Ethoxyethyl(Meth)Acrylat, 2'-Ethoxy-2-Ethoxyethyl(Meth)Acrylat, Polyethylenglycolmono(Meth)Acrylat, Polypropylenglycolmono(Meth)Acrylat, Polytetramethylenglycolmono(Meth)Acrylat. Bevorzugt sind Hydroxyethyl(Meth)Acrylat, Hydroxypropyl(Meth)Acrylat, Hydroxybutyl(Meth)Acrylat, Glycerinmono(Meth)Acrylat und Polyethylenglycolmono(Meth)Acrylat.

Geeignete Monomere b) mit zwei oder mehr Acrylat- oder Methacrylatgruppen sind beispielsweise 1,3-Propandioldi(meth)acrylat; 1,3-Butandioldi(meth)acrylat; 1,4-Butandioldi(meth)acrylat; 1,5-Pentandioldi(meth)acrylat; Neopentylglykoldi(meth)acrylat; 1,6-Hexandioldimethacrylat; 1,9-Nonandioldi(meth)acrylat; 1,10-Decandioldi(meth)acrylat; 1,12-Dodecandioldi(meth)acrylat; Glycerindi(Meth)Acrylat, Ethylenglykoldi(meth)acrylat; Diethylenglykoldi(meth)acrylat; Triethylenglykoldi(meth)acrylat; Propoxyliertes (2) Neopentylglykoldi(meth)acrylat; Bisphenol-A-di(meth)acrylat; Bisphenol-A-glyceroldi(meth)acrylat (BisGMA); Ethoxyliertes Bisphenol-A-Di(meth)acrylat; Propoxyliertes Bisphenol-A-Di(meth)acrylat; Diurethandi(meth)acrylat (UDMA); Tricyclo[5.2.1.0]decan-dimethanoldi(meth)acrylate; Bis[2-(2-methylacryl-amino)-ethoxycarbonyl]-hexamethylendiamin; Trimethylolpropan-tri(meth)acrylat; Di-Trimethylolpropan-tetra(meth)acrylat; Di-Pentaerythritol-penta(meth)-acrylat; Di-Pentaerythritolhexa(meth)acrylat; die polyalicyclischen Strukturen, die in EP 2,436,363; EP 2,436,366; EP 2,436,388; EP 2,450,025; US 8,697,772 ; US 8, 669,302; WO 2013/023138 beschrieben werden. Bevorzugt sind Bisphenol-A-di(meth)acrylat; Bisphenol-A-glyceroldi(meth)acrylat (BisGMA); Ethoxyliertes Bisphenol-A-Di(meth)acrylat; Propoxyliertes Bisphenol-A-Di(meth)acrylat; Diurethandi(meth)acrylat (UDMA) und Tricyclo[5.2.1.0]decan-dimethanoldi(meth)acrylate.

In einer Ausführungsform enthält das erfindungsgemäße Material bevorzugt Photoinitiatoren bzw. Photoinitiator-Systeme.

Solche, bevorzugt für den Wellenbereich von 390 nm bis 500 nm optimierte, Photoinitiatoren bzw. Photoinitiator-Systeme sind dem Fachmann bekannt. Solche Initiatoren bzw. Initiator-Systeme enthalten beispielsweise Campherchinon, 1-Phenyl propan-1,2 dion, Benzildiacetyl, Benzyldimethylketal, Benzyldiethylketal, Benzyldi(2-methoxyethyl)ketal, Anthrachinon, 1-Chloranthrachinon, 1-Chloranthrachinon, 1,2-Benzanthrachinon, 1-Hydroxyanthrachinon, 1-Methylanthrachinon, 2-Ethylanthrachinon, 1-Bromanthrachinon, Thioxanthon, 2-Isopropylthioxanthon, 2-Nitrothioxanthon, 2-Methylthioxanthon, 2,4-Dimethylthioxanthon, 2,4-Diethylthioxanthon, 2,4-Diisopropylthioxanthon, 2-Chlor-7-trifluormethylthioxanthon, Thioxanthon-10,10-dioxid, Thioxanthon-10-oxid, Benzoinmethylether, Benzoinethylether, Benzoinisopropylether, Benzoinisobutylether, Benzophenon, Bis(4-dimethylamino-phenyl)keton, 4,4'-Bisdiethylaminobenzophenon, Acylphosphinoxide wie 2,4,6 Trimethylbenzoyl)diphenylphosphinoxid, Bis(2,4,6-trimethylbenzoyl)-phenylphosphinoxid, Benzoyltrimethylgermanium, Dibenzoyldiethylgermanium, und Diaryliodoniumsalze, wie Diphenyliodoniumhexafluorophosphat, Diphenyliodoniumhexafluoroantimonat, Bis(4-bromophenyl)iodoniumtriflat, Bis(4-tert-butylphenyl)iodoniumhexafluorophosphat, Bis(4-fluorophenyl)iodoniumtriflate, Bis(4-methylphenyl)iodoniumhexafluorophosphat, (2-Bromophenyl)(2,4,6-trimethylphenyl)iodoniumtriflat, 3-Bromophenyl)(2,4,6-trimethylphenyl)iodoniumtriflat, (2-Methylphenyl)(2,4,6-trimethylphenyl)iodoniumtriflat, (3-Methylphenyl)(2,4,6-trimethylphenyl)iodoniumtriflat, (4-Methylphenyl)(2,4,6-trimethylphenyl)iodoniumtriflat, (4-Nitrophe-nyl)phenyliodoniumtriflat, (4-Nitrophenyl)(2,4,6-trimethylphenyl)iodoniumtriflat, Phenyl[3-(trifluoromethyl)phenyl]iodoniumtriflat und [3-(Trifluoromethyl)phenyl](2,4,6-trimethylphenyl)iodoniumtriflat. Bevorzugt sind Systeme, die Diphenyliodoniumhexafluorophosphat oder Campherchinon enthalten.

Photoinitiator-Systeme können bevorzugt Elektronendonoren als Co-Initiator enthalten. Besonders bevorzugt als Co-Initiatoren sind beispielsweise tertiäre aromatische oder aliphatische Amine wie N,N-Dimethylaminoethylmethacrylat oder Aminobenzoate, wie 2-Ethylhexyl-4-(dimethylamino)benzoat und Ethyl(N,N-dimethylamino)benzoat. Besonders bevorzugte Systeme sind Kombinationen von Campherchinon mit Dimethylaminobenzoaten. Weitere besonders bevorzugte System sind die in PCT/EP2015/053303 (Böttcher, Henrik)genannten Initiatorsysteme.

In einer weiteren bevorzugten Ausführungsform, die mehrkomponentig, bevorzugt zweikomponentig ist, enthält das erfindungsgemäße Material ein aktiviertes Initiatorsystem für radikalische Polymerisationsreaktionen, das bevorzugt bei Raumtemperatur (23°C) und noch bevorzugter bei Körpertemperatur (37°C) aktivierbar ist. Aktivierte Initiatorsysteme bestehen mindestens aus einem Aktivator und einem Initiator. Aktivator und Initiator sind zur Vermeidung von vorzeitiger Erhärtung in getrennt gelagerten Komponenten des Materials enthalten. Die Materialkomponenten werden unmittelbar vor der Anwendung miteinander gemischt. In der Mischung führt die Reaktion der Aktivatoren mit den Initiatoren zur Initiierung der radikalischen Polymerisation der Monomere und damit zur Erhärtung des Materials.

In einer Ausführungsform ist das aktivierte Initiatorsystem ein RedOX-Initiatorsystem.
Als ein bevorzugtes RedOx-Inititorsystem ist das Amin-Peroxid System bekannt. In dieser Ausführungsform besteht das RedOx-Inititorsystem mindestens aus einem Amin als Reduktionsmittel und einem Peroxid als Oxidationsmittel, wie in DE 10 2009 005 480 (Kohro, Y.), EP 1 444 972 (Finger, W.), US 7,820,733 (Ohara, Y.) und US 7,214,276 (Qian, X.) beschrieben. Geeignete Amine sind aliphatische tertiäre Amine, wie beispielsweise N,N-Dimethylaminoethylmethacrylat, Triethylamin, Tributylamin, Triallylamin, Triethanolamin, aromatische sekundäre und insbesondere aromatische tertiäre Amine, wie z.B. N,N-Dimethylanilin, N,N-Di(2-hydroxyethyl)-3,5-dimethylanilin, N-Methyl-N-(2-methylcarbamoyloxypropyl)-3,5-dimethylanilin, N,N-Dimethyl-p-tert-butylanilin, N,N-Dimethyl-p-toluidin, N,N-Ethyl-p-toluidin, N,N-Di(2-hydroxyethyl)-p-toluidin, N,N-Di(hydroxymethyl)-p-toluidin, N-Methylp-toluidin, N,N-Dimethyl-sym.-xylidin, Phenylmorpholin, Methyl-4-Dimethylaminobenzoat, Ethyl-4-Dimethylaminobenzoat, Isoamyl-4-Dimethylaminobenzoat und dergleichen. Bevorzugt sind aromatische tertiäre Amine, wie N,N-Dimethyl-p-toluidin, N,N-Di(hydroxymethyl)-p-toluidin, N,N-Dimethyl-sym.-xylidin und N,N-Dimethyl-p-tert-butylanilin.
Als geeignete Peroxide sind nicht allumfassend folgende Verbindungen genannt, Dibenzoylperoxid, 4,4'-Dichlordibenzoylperoxid, 2,4-Dichlordibenzoylperoxid, Dilauroylperoxid, tert-Butylperoxid, tert-Butylperoxybenzoat, Methylethylketonperoxid und tertiäre Hydroperoxide wie, t-Butylhydroperoxid, t-Amylhydroperoxid, p-Diisopropylbenzolhydroperoxid, Cumolhydroperoxid, Pinanhydroperoxid, p-Methanhydroperoxid, und 1,1,3,3-Tetramethylbutylhydroperoxid, 2,5-Dimethylhexan-2,5-dihydroperoxid. In bestimmten Ausführungsformen können auch anorganische Peroxide, als Beispiel seien Natriumperoxodisulfat, Kaliumperoxodisulfat, Ammoniumperoxodisulfat genannt, verwendet werden wie in EP 1 878 428 (Tokui, H.). Besonders bevorzugt sind Diacylperoxide, wie Dibenzoylperoxid und Dilauroylperoxid.

In einer weiteren bevorzugten Ausführungsform ist das aktivierte Initiatorsystem ein RedOx-Initiatorsystem bestehend aus einem substituiertem Thioharnstoff als Reduktionsmittel und einem Hydroperoxid als Oxidationsmittel wie in WO 03/057792 (Mitra, S.) und WO 2008/134024 (Liu, H.) genannt. Substituierte Thioharnstoffe beinhalteten, sind aber nicht beschränkt auf, die Verbindungen Benzoylthioharnstoff, 1-(2-Pyridyl)-2-thioharnstoff, 1-Acetyl-2-thioharnstoff und 1-(2-Tetrahydrofurfuryl)-2-thioharnstoff oder polymerisierbare Thioharnstoffe, wie 1-Allylthioharnstoff, 1,1-Diallylthioharnstoff, 1,3-Diallylthioharnstoff, 1-Allyl-3-(2-hydroxyethyl)-2-thioharnstoff, (Meth)acryloxyalkylthioharnstoff, 1-Allyl-3-methylthioharnstoff. Besonders geeignet sind Allylthioharnstoff-Derivate, 1-(2-Pyridyl)-2-thioharnstoff und 1-(2-Tetrahydrofurfuryl)-2-thioharnstoff.
In dieser Ausführungsform sind als Oxidationsmittel die folgenden Hydroperoxide geeignet, Cumolhydroperoxid, t-Butylhydroperoxid, t-Amylhydroperoxid, p-Diisopropylbenzolhydroperoxid, Pinanhydroperoxid, p-Methanhydroperoxide, und 1,1,3,3-Tetramethylbutylhydroperoxid. Besonders geeignet sind Cumolhydroperoxid und t-Amylhydroperoxid.
Optional kann diesem System ein saurer Promotor, wie z. B. Methacrylsäure, hinzugegeben werden wie in US 2003/134933 (Jin, S.) beschrieben.

Eine weitere günstige Ausführungsform ist ein System, bestehend aus Ascorbinsäure bzw. einem Derivat der Ascorbinsäure und einem Oxidationsmittel, beschrieben in US 5,501,727 (Wang, B.) und EP 2 371 346 (Yarimizu, H.). Geeignete Oxidationsmittel sind ausgewählt aus den Stoffgruppen der Peroxodisulfate, wie Natrium-, Kalium-, Ammonium- und Alkylammonium-Peroxodisulfat, der Peroxide, wie beispielsweise Dibenzoylperoxid, Stearylperoxid, Succinsäureperoxid, und der Hydroperoxide, wie Cumolhydroperoxid, tert-Butylhydroperoxid, tert-Amylhydroperoxid und 2,5-Dihydroperoxy-2,5-dimethylhexan. Besonders geeignet als Oxidationsmittel sind Kaliumperoxodisulfat und tert-Butylhydroperoxid sowie t-Amylhydroperoxid.
Als Beispiele für Ascorbinsäure-Verbindungen sind seien L(+) Ascorbinsäure, Dehydroascorbinsäure, Isoascorbinsäure, Tolyl-L-ascorbinsäure, 2,6-di-o-Palmitoyl-L-ascorbinsäure, D-Araboascorbinsäure, L(+)Natriumascorbat, Natriumisoascorbat und L(+)Calciumascorbat genannt Besonders geeignet sind Ammonium, Kalium- oder Natriumisoascorbat.

Ebenfalls geeignet ist das Sulfinat-Peroxid-System, wie in DE 698 29 259 (Yamamoto, T.), EP 2 110 392 (Takei, M.), WO 2005/035590 (Kalgutar, R.) und EP 1 878 418 (Tokui, H.) beschrieben. Als Beispiel für die in dieser Ausführungsform bevorzugten aromatischen Sulfinate sind die Erdalkali-, Alkali- und Ammonium-Salze aromatischer Sulfinsäuren, wie zum Beispiel der Benzolsulfinsäure, der p-Toluolsulfinsäure, o-Toluolsulfinsäure, Ethylbenzolsulfinsäure, Decylbenzolsulfinsäure, Dodecylbenzolsulfinsäure, 2,4,6-Trimethylbenzolsulfinsäure, 2,4,6-Triisopropylbenzolsulfinsäure, Chlorbenzolsulfinsäure oder Naphthalensulfinsäure, genannt. Besonders geeignet sind die Benzolsulfinate und die Alkylderivate, wie Toluolsulfinsäure, davon.
Illustrative Beispiele für verwendete Peroxide schließen organische Peroxide wie Diacylperoxide, Peroxyester, Dialkylperoxide, Peroxyketale, Ketonperoxide und Hydroperoxide ein.
Spezifische Beispiele für Diacylperoxide sind Diacetyldiperoxid, Dibenzoylperoxid, 4,4'-Dichlordibenzoylperoxid, 2,4-Dichlorodibenzoylperoxid, p,p'-Dimethoxydibenzoylperoxid, p,p'-Dimethyldibenzoylperoxid, p,p'-Dinitrodibenzoylperoxid und m-Ditoluoylperoxid.
Spezifische Beispiele für Peroxyester sind t-Butylperoxybenzoat, Bis-t-butylperoxyisophthalat, 2,5-Dimethyl-2,5-bis(benzoylperoxy)hexan, t-Butylperoxy-2-ethylhexanoat und t-butylperoxyisopropylcarbonat.
Spezifische Beispiele für Dialkylperoxide sind Dicumylperoxid, Dipropylperoxid, Dibutylperoxid, Di-t-butylperoxid, Dicaprylperoxid und Dilaurylperoxid.
Spezifische Beispiele für Peroxyketale sind 1,1-Bis(t-butylperoxy)3,3,5-trimethylcyclohexan, 1,1-Bis(t-butylperoxy)cyclohexan und 1,1-Bis(t-hexylperoxy)cyclohexan.

Spezifische Beispiele für Ketonperoxide sind Methylethylke-tonperoxid, Cyclohexanonperoxid und Methylacetoacetatperoxid. Spezifische Beispiele für Hydroperoxide sind Cumolhydroperoxid, tert-Butylhydroperoxid und tert-Amylhydroperoxid, 2,5-Dihydroperoxy-2,5-dimethylhexan.
Auch anorganische Peroxide, wie beispielsweise Natriumperoxid, Kaliumperoxid, Aluminiumperoxid, Ammoniumperoxid, Ammoniumpersulfat und Kaliumpersulfat, sind in bestimmten Ausführungsformen günstig.
Besonders geeignet sind Dibenzoylperoxid und Natrium- oder Kaliumperoxodisulfat.

In einer ebenso bevorzugten mehrkomponentigen, bevorzugt zweikomponentigen Ausführungsform enthält das erfindungsgemäße Material ein Initiatorsystem basierend auf CH-aciden Komponenten, bspw. auf der Basis von Barbitursäure- oder Thiobarbitursäurederivaten, wie in H. Bredereck et al.,Makromol. Chem. 92, 70 (1966) und US 5376691 (May. U.) oder, in modifizierter Form, in EP 2 512 400 (Neffgen, St.) oder in EP 1 194 110 (Soglowek, W.) beschrieben.

Ebenfalls geeignete Initiatorsysteme basierend auf Salzen CH-acider Verbindungen, sind in EP 1 872 767 (Lück, R.) beschrieben. Diese Initiatorsysteme haben den Vorteil, dass das Salz der CH-aciden Verbindung in Materialkomponenten enthalten sein können, die überdies radikalisch polymerisierbare Monomere umfassen, ohne dass es zu einer Selbsterhärtung dieser Materialkomponenten kommt. In einem Zweikomponentenmaterial ist in einer solchen Ausführungsform das Salz der CH-aciden Verbindung zwingend in einer Komponente enthalten, die keine Säure und insbesondere nicht die Verbindungen nach Formel I enthält. Initiatorsysteme, die Salze CH-acider Verbindungen enthalten, können in bestimmten Ausführungsformen der vorliegenden Erfindung kombiniert mit RedOx-Initiatorsystemen zum Einsatz kommen wie in EP 2 198 824 (Neffgen, St.) beschrieben.

Weitere geeignete Initiatorsysteme enthalten Borverbindungen zur Initiierung der radikalischen Polymerisation so wie die in JP2006-111584 genannten Borate, die nach chemischer Aktivierung durch Säuren die radikalische Polymerisation initiieren. Andere geeignete Initiatorsysteme, die Borverbindungen enthalten sind in EP1489103 (Tomikawa, T.) beschrieben. Diese in Verbindung mit Luftsauerstoff die radikalische Polymerisation initiierenden Bor-Verbindungen sind beispielsweise Trialkylborane, Alkoxyalkylborane, Dialkylborane und partiell oxidierte Trialkylborane. Als Beispiele für Alkylborane seien Triethylboran, Tripropylboran, Triisopropylboran, Tributylboran, Tri-sec-butylboran, Triisobutylboran, Tripentylboran, Trihexylboran, Trioctylboran, Tridecylboran, Tricyclpentylboran oder Tricyclhexylboran genannt. Als Beispiele für Alkoxyalkylborane seien Butoxydibutylboran, für Dialkylborane 9-Borabicyclo[3.3.1]nonan und partiell oxidierte Borverbindungen partiell oxidiertes Tributylboran erwähnt.
Es kann bevorzugt sein, dass das erfindungsgemäße Material eine Kombination aus einem oder mehreren aktiviertem Initiatorsystemen mit einem oder mehreren Fotoinitiatorsystemen enthält. In diesen Fällen kann das Material durch Lichteinwirkung gehärtet werden, härtet aber auch in unbelichteten Bereichen vollständig aus.

Bei einer bevorzugten Ausführungsform der Erfindung beträgt der Anteil radikalisch polymerisierbarer Monomere an der Gesamtmasse des Dentalmaterials 10-99,98 Gew.-%. Der verbleibende Rest umfasst die Initiatoren sowie unten näher beschriebene fakultative Bestandteile und Lösungsmittel. Bevorzugt ist eine gegebenenfalls geringe Menge Wasser enthalten, um die Reaktion zwischen der Säuregruppe und dem Hydroxylapatit der Zahnsubstanz unter bestimmten Bedingungen zu beschleunigen. Gegebenenfalls kann diese Reaktion bei einem wasserfreien Dentalmaterial durch die Feuchtigkeit in der Mundhöhle ermöglicht werden. Erfindungsgemäße Zusammensetzungen haben auch den Vorteil, dass auch bei Wasser-Konzentrationen von niedriger als 30 Gewichtsanteilen sich homogene Lösungen der Harze und des Initiator-Systems bilden.

Vorzugsweise beträgt der Anteil radikalisch polymerisierbarer Monomere der Merkmalsgruppe a. an der Gesamtmenge radikalisch polymerisierbarer Monomere 1 bis 50 Gewichtsteile, weiter vorzugsweise 5 bis 50 Gewichtsteile.

Vorzugsweise beträgt der Anteil radikalisch polymerisierbarer Monomere der Merkmalsgruppe b. an der Gesamtmenge radikalisch polymerisierbarer Monomere 99 bis 50 Gewichtsteile, weiter vorzugsweise 95 bis 50 Gewichtsteile.

Gemäß einer weiteren bevorzugten Ausführungsform der Erfindung sind ein oder mehrere, bevorzugt alle Substituenten der Formel 1 ausgewählt sind aus der Gruppe bestehend aus:
R1= H
R2 = C1-C6-Alkyl
R3 = H
A = C8-C14-Alkylen.

Erfindungsgemäß sind Acrylamido-Bisphosphonsäuren (R1=H) gegenüber Methacrylamido-Bisphosphonsäuren (R1=CH3) weiter bevorzugt.

Der Anteil des wenigstens einen Initiators für die radikalische Polymerisation an der Gesamtmasse des Dentalmaterials beträgt vorzugsweise 0,01-10 Gew.-%, weiter vorzugsweise 0,01-5 Gew.-%.

Bevorzugt als Initiator oder Initiator-System ist ein Photoinitiator-System, weiter bevorzugt ein Photoinitiator-System für den Wellenlängenbereich zwischen 390 und 500 nm, wie oben bereits näher beschrieben.

In einer weiteren Variante der Erfindung enthält das Dentalmaterial 0-90 Gew.-% mindestens eines Lösungsmittels ausgewählt aus der Gruppe bestehend aus Wasser und organisches Lösungsmittel.

Bevorzugte Untergrenzen für den Wasseranteil sind 0,0; 0,1; 1 oder 5 Gew.-%, bevorzugte Obergrenzen sind 20 oder 15 Gew.-%.

Bevorzugte Untergrenzen für den Anteil organischer Lösungsmittel sind 0,0; 0,1; 1 oder 5 Gew.-%, bevorzugte Obergrenzen sind 90, 50 oder 20 Gew.-%.

Organische Lösungsmittel sind bevorzugt ausgewählt aus der Gruppe bestehend aus bei Raumtemperatur (23 °C) flüchtigen Lösungsmitteln mit einer nach DIN 153170 bestimmten Verdunstungszahl < 80. Geeignete organische Lösungsmittel sind polare, bevorzugt polar protische Lösungsmittel, beispielsweise Aceton, Propanole, Butanole, insbesondere Ethanol.

Gemäß einer weiteren Variante der Erfindung enthält das Dentalmaterial 0,01-10 Gew.-% dentalüblicher Additive, bevorzugt Additive ausgewählt aus der Gruppe bestehend aus Inhibitoren, Stabilisatoren, Beschleunigern, Farbmitteln, Fluoridierungsmitteln, Remineralisierungsmitteln, Röntgenopakern und zusätzlichen Filmbildnern.

Ein erfindungsgemäßes Dentalmaterial kann Füllstoffe enthalten, insbesondere dann, wenn es beispielsweise als Komposit, Zement oder dergleichen ausgebildet oder für eine solche Verwendung vorgesehen ist.

Geeignete Füllstoffe sind beispielsweise, Glaspulver, Glaskeramikpulver, Quarzpulver, Metalloxide, Metallhydroxide, sphärische Füllstoffe wie z.B. in der DE-PS 3247800 beschrieben, amorphe Cluster-Füllstoffe wie z.B. in der WO 01/30306 beschrieben oder eine Mischung aus diesen Füllstoffen, insbesondere Bariumsi-likatgläser, Strontiumsilikatgläser, Borataluminosilikatgläser, Phosphataluminosilikatgläser, Fluoroaluminosilikatgläser, Calciumsilikate, Zirkonsilikate, Natriumaluminiumsilikate, Schichtsilikate, Bentonite, Zeolithe einschließlich der Molekularsiebe, die Oxide sowie die Hydroxide der Alkali- und der Erdalkalimetalle sowie Apatit.

Bevorzugt ist eine Untergrenze des Anteils der Füllstoffe an der Gesamtmasse des Dentalmaterials 0,0; 0,1 oder 1 Gew.-%. Eine bevorzugte Obergrenze ist 90, 80, 65 oder 45 Gew.-%.

In einer Variante der Erfindung sind die Füllstoffe bevorzugt ausgewählt aus der Gruppe bestehend aus:
- Dentalgläsern, bevorzugt Dentalgläser mit einer mittleren Partikelgröße zwischen 200 nm und 50 µm, die weiterhin bevorzugt Acrylat- und/oder Methacrylatgruppen an ihrer Oberfläche aufweisen;
- pyrogenen Kieselsäuren;
- nass gefällten Kieselsäuren;
- Nanofüllstoffen mit einer mittleren Partikelgröße < 100 nm, die bevorzugt nicht aggregiert sind.
- Nanofüllstoffe mit einer mittleren Partikelgröße < 100 nm, die bevorzugt nicht aggregiert und nicht agglomeriert sind

Gemäß einer weiteren bevorzugten Variante der Erfindung weisen die radikalisch polymerisierbaren Monomere der Merkmalsgruppe b. keine Acrylamid- und/oder Methacrylamidgruppen auf; weiter bevorzugt keine radikalisch polymerisierbare Gruppe, die verschieden ist von Acrylat- und/oder Methacrylatgruppen. Mischungen aus Komponenten, die Acrylat- und/oder Methacrylatgruppen enthalten, zeichnen zum einen durch eine gute Reaktivität für radikalische Polymerisationen und zum anderen durch eine breite Verfügbarkeit aus.

Gemäß einer weiteren bevorzugten Variante der Erfindung weisen die radikalisch polymerisierbaren Monomere der Merkmalsgruppe b. keine Säuregruppen und/oder keine Polyoxyalkylengruppen mit mehr als vier Oxyalkyleneinheiten auf. Eine solche Zusammensetzung weist im ausgehärteten Zustand in der bevorzugten Ausführungsform eine vorteilhafte geringe Wasseraufnahme bzw. Wasserlöslichkeit auf.

Das erfindungsgemäße Dentalmaterial kann formuliert sein als ein Material ausgewählt aus der Gruppe bestehend aus Bondings, Adhäsiven, Fissurenversieglern, Befestigungszementen, Füllungsmaterialien und Kompositen.

Ebenfalls Gegenstand der Erfindung ist die Verwendung eines erfindungsgemäßen Dentalmaterials für die im vorstehenden Absatz genannten Zwecke.

Ausführungsbeispiele der Erfindung werden nachfolgend erläutert.

### Herstellungsbeispiele

### 11-Bromundecanbisphosphonsäuretetraethylester (1)

Unter Stickstoff wurden 1,4 g Natriumhydrid (60 % in Mineralöl, 35 mmol) in 15 ml Tetrahydrofuran (THF) suspendiert. Die Suspension wurde im Eisbad auf 0-5°C heruntergekühlt und 8,5 g (30 mmol) Tetraethylmethylenbisphosphonsäureester wurden hinzugetropft. 44,4 g (148 mmol) 1,10-Dibromdecan wurden in 40 ml THF gelöst und dem Reaktionsgemisch zugesetzt. Die Lösung wurde 72 h bei RT gerührt und dann mit 30 ml wässriger Natriumhydrogencarbonat-Lösung (0,1 mol/l) versetzt. Anschließend wurde das THF abrotiert und die wässrige Phase 2 mal mit Toluol ausgeschüttelt. Die vereinigten organischen Phasen wurden einrotiert. Mit einer Säulen-Filtration (SiO₂, 1. Eluent: Heptan/Ethylacetat 1:1, v/v; 2. Eluent: Ethanol) wurde Dibromdecan abgetrennt. Das Rohprodukt wurde mit Hilfe einer Flashchromatographie aufgereinigt (SiO₂; Ethylacetat/Ethanol, 95:5, v/v). Es wurde ein gelbliches Öl erhalten (Ausbeute: 65 %).
¹H-NMR (CDCl₃, 300 MHz): δ = 1,02-1,38(m, 12 H, CH₂), 1,34(t, ³J_{HH} = 7 Hz, 12H, POCH₂CH₃), 1,38-1,48(m, 2H, CH₂), 1,48-1,62(m, 2H, CH₂), 1,72-2,02 (m, 2H, CH₂), 2,27(tt, ²J_{HP} = 24 Hz; ³J_{HH} = 6 Hz, 1H, CHP), 3,41 (t, ³J_{HH} = 6 Hz, 2H, CH₂Br), 4, 03-4, 26(m, 8H, POCH₂CH₃).

### 6-Bromhexanbisphosphonsäuretetraethylester (2)

Die Herstellung erfolgte analog des 11-Bromundecanbisphosphonsäure-tetraethylesters unter Verwendung von 39,5 g (172 mmol) 1,5-Dibrompentan Mit einer Säulen-Filtration (SiO₂, 1. Eluent: Heptan/Ethylacetat 1:1, v/v; 2. Eluent: Ethanol) wurde auch hier Dibrompentan abgetrennt. Das Rohprodukt, welches als gelbliches Öl anfällt, wurde ohne weitere Reinigung für den nächsten Schritt eingesetzt.
¹H-NMR (CDCl₃, 300 MHz): δ = 1, 02-1, 38 (m, 2 H, CH₂), 1,34(t, ³J_{HH} = 7 Hz, 12H, POCH₂CH₃), 1,38-1,48 (m, 2H, CH₂), 1,48-1,62(m, 2H, CH₂), 1,72-2,02 (m, 2H, CH₂), 2,27(tt, ²J_{HP} = 24 Hz; ³J_{HH} = 6 Hz, 1H, CHP), 3,41 (t, ³J_{HH} = 6 Hz, 2H, CH₂Br), 4,05-4,26(m, 8H, POCH₂CH₃).

### 11-Aminoundecanbisphosphonsäuretetraethylester (3)

1,6 g Kaliumphthalimid (8,6 mmol) wurden mit 15 ml Aceton versetzt. Der Lösung wurden 3,7 g (7,3 mmol) 11-Bromundecanbisphosphonsäuretetraethylester (1) zugesetzt und in der Mikrowelle 10 h bei 70°C gerührt (Mikrowelle: Discover CEM, Leistung: 100 W). Danach wurde das Reaktionsgemisch filtriert und der Rückstand mit Aceton gewaschen. Das Filtrat und die Aceton-Phase wurden vereinigt und einrotiert. Der Rückstand wurde in 20 ml Dichlormethan gelöst und die organische Phase mit 15 ml Wasser ausgeschüttelt. Die Dichlormethanphase wurde anschließend einrotiert und unter Vakuum getrocknet. Das Zwischenprodukt (4,2 g) wurde in 170 ml eines Wasser-(Propan-2-ol)-Gemisches (1:6, v/v) gelöst. Dieser Lösung wurden 1,4 g (37 mmol) Natriumborhydrid hinzugesetzt. Nach 24 h Rühren bei Raumtemperatur (RT) wurden 17 ml Essigsäure hinzugegeben und die Lösung 2 h bei 80°C unter Rückfluss gekocht. Danach wurden 20 ml Wasser zugesetzt und das 2-Propanol abrotiert. Mit konzentrierter Salzsäure wurde die Lösung auf einen pH-Wert von 1 gestellt und mit Toluol ausgeschüttelt. Die Toluol-Phase wurde verworfen und die wässrige Phase mit NaOH alkalisch gestellt (pH = 14). Die alkalische Phase wurde 2x mit Toluol ausgeschüttelt. Die vereinigten organischen Phasen wurden eingeengt und unter Vakuum getrocknet. Es wurden 2,9 g (Ausbeute: 86 %) eines wachsartigen Feststoffes erhalten.
¹H-NMR (MeOD, 300 MHz): δ = 1, 28-1, 44 (m, 12 H, CH₂), 1,34(t, ³J_{HH} = 7,0 Hz, 12H, POCH₂CH₃), 1,50-1,70(m, 4H, CH₂), 1,78-1,99(m, 2H, CH₂), 2,51(tt, ²J_{HP} = 24,3 Hz; ³J_{HH} = 6,0 Hz, 1H, CHP), 2,90(t, ³J_{HH} = 7,5 Hz, 2H, CH₂NH₂), 4,07-4,27 (m, 8H, POCH₂CH₃).

### 11-(Propylamino)undecanbisphosphonsäuretetraethylester (4)

2,5 g (5 mmol) 11-Bromundecanbisphosphonsäuretetraethylester (1) wurden mit 8,2 ml n-Propylamin (100 mmol) versetzt. Diese Lösung wurde in der Mikrowelle (Mikrowelle: Discover CEM, Leistung: 150 W) 5 min bei 80°C gerührt. Danach wurde das überschüssige Propylamin abrotiert und der Rückstand in 20 ml NaOH-Lösung (0,1 mol/l) gelöst. Die wässrige Phase wurde dreimal mit Toluol ausgeschüttelt. Die vereinigten organischen Phasen wurden eingeengt und unter Vakuum getrocknet. Es wurde ein gelbliches Öl erhalten (Ausbeute: 92 %), welches ohne weitere Aufarbeitung für die Acylierung eingesetzt wurde.
¹H-NMR (CDCl₃, 300 MHz): δ = 0,92(t, ³J_{HH} = 7,4 Hz, 3H, CH₃), 1,20-1,40(m, 12 H, CH₂), 1,34(t, ³J_{HH} = 7,0 Hz, 12H, POCH₂CH₃), 1,42-1,62(m, 6H, CH₂), 1,78-2,03(m, 2H, CH₂), 2,27(tt, ²J_{HP} = 24,3 Hz; ³J_{HH} = 6,0 Hz, 1H, CHP), 2,53-2,64(m, 4H, CH₂NH), 4,10-4,26 (m, 8H, POCH₂CH₃).

### 6-(Propylamino)hexanbisphosphonsäuretetraethylester (5)

8,5 g (19 mmol) 6-Bromhexanbisphosphonsäuretetraethylester (2) wurden mit 32 ml n-Propylamin (380 mmol) versetzt. Diese Lösung wurde in der Mikrowelle (Mikrowelle: Discover CEM, Leistung: 150 W) 10 min bei 100°C gerührt. Danach wurde das überschüssige Propylamin abrotiert und der Rückstand in 20 ml NaOH-Lösung (0,1 mol/l) gelöst. Die wässrige Phase wurde dreimal mit Toluol ausgeschüttelt. Die vereinigten organischen Phasen wurden eingeengt und unter Vakuum getrocknet. Mit einer Säulen-Filtration (SiO₂, 1. Eluent: Ethylacetat/Ethanol 4:1, v/v; 2. Eluent: Ethanol) wurde das Rohprodukt aufgereinigt. Es wurde ein farbloses Öl erhalten (Ausbeute: 24 %).
¹H-NMR (CDCl₃, 300 MHz) : δ = 0,96(t, ³J_{HH} = 7,4 Hz, 3H, CH₃), 1,30-1,48(m, 2 H, CH₂), 1,34(t, ³J_{HH} = 7,0 Hz, 12H, POCH₂CH₃), 1,50-1,80 (m, 6H, CH₂), 1,82-2,05(m, 2H, CH₂), 2,29(tt, ²J_{HP} = 24 Hz; ³J_{HH} = 6,0 Hz, 1H, CHP), 2,66-2,80 (m, 4H, CH₂NH), 4,10-4,24 (m, 8H, POCH₂CH₃).

### 11-Acrylamidoundecanbisphosphonsäuretetraethylester (6)

2 g (4,5 mmol) 11-Aminoundecanbisphosphonsäuretetraethylester (3) und 1,2 ml (9 mmol) Triethylamin wurden in 20 ml getrocknetem Dichlormethan gelöst. Bei 0-5°C wurde unter Rühren 0,55 ml (6,8 mmol) Acrylsäurechlorid, welches in 10 ml Dichlormethan gelöst war, hinzugegeben. Nach 3 h Rühren bei RT wurde die Lösung filtriert und die Dichlormethanphase eingeengt. Das Rohprodukt wurde mit Hilfe einer Flashchromatographie aufgereinigt (SiO₂; Ethylacetat/Ethanol, 80:20, v/v). Es wurde ein farbloses Öl erhalten (Ausbeute: 35 %).
¹H-NMR (MeOD, 300 MHz): δ = 1,25-1,46(m, 12 H, CH₂), 1,34(t, ³J_{HH} = 7,0 Hz, 12H, POCH₂CH₃), 1,46-1,66(m, 4H, CH₂), 1,78-1,99(m, 2H, CH₂), 2,51(tt, ²J_{HP} = 24,3 Hz; ³J_{HH} = 6,0 Hz, 1H, CHP), 3,23(m, 2H, CH₂N), 4,09-4,24 (m, 8H, POCH₂CH₃), 5,63 (dd, 1H, CH₂=CH), 6,20(d, 1H, CH₂=CH), 6,22 (d, 1H, CH₂=CH).

### 11-(N-Propylacrylamido)undecanbisphosphonsäuretetraethylester (7)

2,4 g (5 mmol) 11-(Propylamino)undecanbisphosphonsäuretetraethylester (4) und 1,1 ml (8 mmol) Triethylamin wurden in 30 ml getrocknetem Dichlormethan gelöst. Bei 0-5°C wurde unter Rühren 0,6 ml (7,5 mmol) Acrylsäurechlorid, welches in 10 ml Dichlormethan gelöst war, hinzugegeben. Nach 3 h Rühren bei RT wurde die Lösung filtriert und die Dichlormethan-Phase mit Wasser ausgeschüttelt. Die wässrige Phase wurde anschließend mit Dichlormethan gegengeschüttelt. Die vereinigten organischen Phasen wurden einrotiert und der Rückstand mit Hilfe einer Flashchromatographie aufgereinigt (SiO₂; Ethylacetat/Ethanol, 80:20, v/v). Es wurde ein gelbes Öl erhalten (Ausbeute: 78 %).
¹H-NMR (CDCl₃, 300 MHz) : δ = 0,88(t, ³J_{HH} = 7,4 Hz, 3H, CH₃), 1,13-1,40(m, 12 H, CH₂), 1,30(t, ³J_{HH} = 7,0 Hz, 12H, POCH₂CH₃), 1,44-1,64(m, 6H, CH₂), 1,76-2,04(m, 2H, CH₂), 2,23(tt, ²J_{HP} = 24,3 Hz; ³J_{HH} = 6,0 Hz, 1H, CHP), 3,16-3,40(m, 4H, CH₂NH), 4,10-4,30 (m, 8H, POCH₂CH₃), 5,62 (dd, ²J_{HH} = 2,4 Hz, ³J_{HH} = 10,2 Hz, 1H, CH₂=CH), 6,30 (dd, ²J_{HH} = 2,4 Hz, ³J_{HH} = 16,8 Hz, 1H, CH₂=CH), 6,51 (dd, ³J_{HH} = 10,2 Hz, ³J_{HH} = 16,8 Hz, 1H, CH₂=CH).

### 6-(N-Propylacrylamido)hexanbisphosphonsäuretetraethylester (8)

2 g (4,5 mmol) 6-(Propylamino)hexanbisphosphonsäuretetraethylester (5) und 1,2 ml (9 mmol) Triethylamin wurden in 20 ml getrocknetem Dichlormethan gelöst. Bei 0-5°C wurde unter Rühren 0,55 ml (6,8 mmol, 1,5 eq) Acrylsäurechlorid, welches in 10 ml Dichlormethan gelöst war, hinzugegeben. Nach 3 h Rühren bei RT wurde die Lösung filtriert und die Dichlormethan-Phase eingeengt. Das Rohprodukt wurde mit Hilfe einer Flashchromatographie aufgereinigt (SiO₂; Ethylacetat/Ethanol, 4:1, v/v). Es wurde ein farbloses Öl erhalten (Ausbeute: 50 %).
¹H-NMR (CDCl₃, 300 MHz): δ = 0,91(t, ³J_{HH} = 7,4 Hz, 3H, CH₃), 1,20-1,4(m, 2H, CH₂), 1,34(t, ³J_{HH} = 7,0 Hz, 12H, POCH₂CH₃), 1,50-1,70(m, 6H, CH₂), 1,80-2,04(m, 2H, CH₂), 2,27(tt, ²J_{HP} = 24,3 Hz; ³J_{HH} = 6,0 Hz, 1H, CHP), 3,20-3,45(m, 4H, CH₂NH), 4,10-4,25 (m, 8H, POCH₂CH₃), 5,70 (dd, ²J_{HH} = 2,4 Hz, ³J_{HH} = 10,2 Hz, 1H, CH₂=CH), 6,30 (dd, ²J_{HH} = 2,4 Hz, ³J_{HH} = 16,8 Hz, 1H, CH₂=CH), 6,51 (dd, ³J_{HH} = 10,2 Hz, ³J_{HH} = 16,8 Hz, 1H, CH₂=CH).

### 11-Acrylamidoundecanbisphosphonsäure (AUbisPS) (9)

0,6 g (1,2 mmol) 11-Acrylamidoundecanbisphosphonsäuretetraethylester (6) wurden in 5 ml getrocknetem Dichlormethan gelöst und mit 0,9 ml (7,2 mmol) Trimethylsilylbromid versetzt. Die Lösung wurde 6 h bei 25°C gerührt und dann unter Vakuum eingeengt. Anschließend wurde zum Rückstand 5 ml Methanol gegeben und die Lösung bei 25°C eine weitere 1 h gerührt. Dann wurde das Lösungsmittel abrotiert, der Rückstand in Natronlauge (1 mol/l) gelöst und die Lösung 24 h bei pH = 13 gerührt. Danach wurde die Lösung durch Zugabe von konz. Salzsäure sauer gestellt (pH = 1) und das ausgefallene Produkt abgetrennt. Abschließend wurde das Produkt bis zur Gewichtskonstanz unter Vakuum getrocknet. Es wurde ein weißer Feststoff erhalten (Ausbeute: 100 %).
¹H-NMR (DMSO, 300 MHz): δ = 1,12-1,33(m, 12 H, CH₂), 1,35-1,57 (m, 4H, CH₂), 1,60-1,84(m, 2H, CH₂), 1,89(tt, ²J_{HP} = 23,0 Hz, 1H, CHP), 3,10(q, ³J_{HH} = 6,6 Hz, 2H, CH₂N), 5,63 (dd, ²J_{HH} = 2,4 Hz, ³J_{HH} = 17,1 Hz, 1H, CH₂=CH) , 6,05 (dd, ²J_{HH} = 2,4 Hz, ³J_{HH} = 17,1 Hz 1H, CH₂=CH) , 6,24 (dd, ³J_{HH} = 10,1 Hz, ³J_{HH} = 17,1 Hz, 1H, CH₂=CH), 8,14(m, 1H, NH).

### 11-(N-Propylacrylamido)undecanbisphosphonsäure (PAUbisPS) (10)

2,0 g (4 mmol) von (7) wurden in 10 ml getrocknetem Dichlormethan gelöst und mit 3,1 ml (24 mmol) Trimethylsilylbromid versetzt. Die Lösung wurde 6 h bei 25°C gerührt und dann unter Vakuum eingeengt. Anschließend wurde der Rückstand mit 10 ml Methanol versetzt und die Lösung bei 25°C eine weitere 1 h gerührt. Dann wurde das Lösungsmittel abrotiert, der Rückstand in Natronlauge (1 mol/l) gelöst und die Lösung 24 h bei pH = 13 gerührt. Danach wurde die Lösung durch Zugabe von konz. HCl sauer gestellt (pH = 1) und das ausgefallene Produkt abgetrennt. Abschließend wurde das Produkt bis zur Gewichtskonstanz unter Vakuum getrocknet. Man erhält einen wachsartigen Stoff (Ausbeute: 99 %) .
¹H-NMR (DMSO, 300 MHz) : δ = 0,83(m, 3H, CH₃), 1,15-1,35(m, 12 H, CH₂), 1,38-1,59(m, 6H, CH₂), 1,62-1,86(m, 2H, CH₂), 1,90(tt, ²J_{HP} = 24,3 Hz; ³J_{HH} = 6,0 Hz, 1H, CHP), 3,16-3,37(m, 4H, CH₂NH), 4,10-4,03 (m, 8H, POCH₂CH₃), 5,63 (dd, ³J_{HH} = 10,2 Hz, 1H, CH₂=CH), 6,10 (dd, ²J_{HH} = 2,4 Hz, ³J_{HH} = 16,8 Hz, 1H, CH₂=CH) , 6,70 (ddd, J_{HH} = 2,6 Hz , ³J_{HH} = 10,2 Hz, ³J_{HH} = 16,8 Hz, 1H, CH₂=CH).

### 6-(N-Propylacrylamido)hexanbisphosphonsäure (PAHbisPS) (11)

0,6 g (1,2 mmol) 6-(N-Propylacrylamido)hexanbisphosphonsäuretetraethylester (8) wurden in 5 ml getrocknetem Dichlormethan gelöst und mit 0,9 ml (7,2 mmol) Trimethylsilylbromid versetzt. Die Lösung wurde 6 h bei 25°C gerührt und dann unter Vakuum eingeengt. Anschließend wurde zum Rückstand 5 ml Methanol gegeben und die Lösung bei 25°C eine weitere 1 h gerührt. Dann wurde das Lösungsmittel abrotiert, der Rückstand in Natronlauge (1 mol/l) gelöst und die Lösung 24 h bei pH = 13 gerührt. Danach wurde die Lösung durch Zugabe von konz. Salzsäure sauer gestellt (pH = 1-2), anschließend mit Hilfe eines Kationenaustauschers (Amberlite IR120 H) aufgereinigt und eingeengt. Abschließend wurde das Produkt bis zur Gewichtskonstanz unter Vakuum getrocknet. Man erhält einen weißen Feststoff (Ausbeute: 100 %).
¹H-NMR (DMSO, 300 MHz): δ = 0,84(m, 3H, CH₃), 1,15-1,27 (m, 2 H, CH₂), 1,38-1,62(m, 6H, CH₂), 1,63-1,86(m, 2H, CH₂), 1,94(tt, ²J_{HP} = 24,3 Hz; ³J_{HH} = 6,0 Hz, 1H, CHP), 3,16-3,30(m, 4H, CH₂NH), 5,63 (dd, ³J_{HH} = 10,2 Hz, 1H, CH₂=CH), 6,10 (dd, ²J_{HH} = 2,4 Hz, ³J_{HH} = 16,8 Hz, 1H, CH₂=CH), 6,70 (ddd, J_{HH} = 2,6 Hz, ³J_{HH} = 10,2 Hz, ³J_{HH} = 16,8 Hz, 1H, CH₂=CH).

### 11-Amino-1-hydroxy-undecanbisphosphonsäure (12)

Unter Stickstoff wurden 22 g (0,11 mol) 11-Aminoundecansäu-re, 9,4 g (0,11 mol) Phosphonsäure und 40 ml Methansulfonsäure in einen Kolben gegeben und unter Rühren auf 65°C erwärmt. Nach Bildung einer homogenen Lösung wurde unter Stickstoff mit Hilfe eines Tropftrichters innerhalb von 30 min 20 ml (0,22 mol) Phosphortrichlorid hinzugegeben. Nach 9 h Rühren bei 65°C wurde die Lösung auf RT abgekühlt und mit 100 ml kaltem Wasser versetzt. Nach 6 h Rühren bei 100°C wurde die Lösung auf RT abgekühlt und durch Zugabe von Wasser zur Fällung gebracht. Der entstehende Niederschlag wurde abfiltriert, mehrfach mit Wasser gewaschen und unter Vakuum getrocknet. Man erhält einen weißen Feststoff (Ausbeute: 84%).
¹H-NMR (D₂O, 300 MHz): δ = 1,05-1,20 (m, 12 H, CH₂), 1,22-1,35 (m, 2 H, CH₂), 1,35-1,45 (m, 2H, CH₂), 1,60-1,80(m, 2H, CH₂), 2,44(t, 2H, CH₂NH).

### 11-(Acrylamido)-1-hydroxy-undecanbisphosphonsäure (AUbisP) (13)

4,2 g (11 mmol) von 12 wurden in 20 ml Wasser gegeben und die Suspension mit NaOH-Lösung (1 mol/l) auf einen der pH-Wert von 11-12 gestellt. Die Lösung wurde in einem Eisbad auf 0-5°C heruntergekühlt und dann tröpfchenweise 1,2 ml (13 mmol) Acrylsäurechlorid hinzugegeben. Die Lösung wurde langsam erwärmt und 3 h bei RT gerührt. Bei Zugabe des Acrylsäurechlorides und während des Rührens wurde der pH-Wert konstant auf 11-12 nachgeregelt. Anschließend wurde die Lösung mit konz HCl auf einen pH-Wert von <2 gestellt. Der entstehende Niederschlag wurde abfiltriert, mehrfach mit Wasser gewaschen und unter Vakuum getrocknet. Man erhält einen weißen Feststoff (Ausbeute: 81%)
¹H-NMR (NaOD/D₂O, 300 MHz) : δ = 1,15-1,30 (m, 12H, CH₂), 1,38-1,50(m, 4H, CH₂), 1,73-1,85(m, 2H, CH₂), 3,15(t, 2H, CH₂N), 5,67 (dd, 1H, CH₂=C), 6,03-6,13 (m, 2H, CH₂=C).

### Beispielzusammensetzungen

Es wurden verschiedene Adhäsivzusammensetzungen hergestellt und deren Haftungseigenschaften auf Zahnschmelz untersucht. Hierzu wurden zunächst die einzelnen Komponenten bei RT (23°C) in einem Glasgefäß unter Ausschluss von die Polymerisation initiierenden Licht eingewogen und verrührt, bis eine homogene Lösung entstanden war. Die neben den hergestellten Verbindungen verwendeten kommerziell erhältlichen Komponenten sind in Tabelle 1 aufgelistet.

**Tabelle 1: Eingesetzte Komponenten**

| | |
|---|---|
| BisGMA | Bisphenol-A-diglycidyldimethacrylat (CAS 001565-94-2) |
| Ethanol | CAS 64-17-5 |
| dest. Wasser | CAS 7732-18-5 |
| HEMA | 2-Hydroxyethylmethacrylat (CAS 868-77-9) |
| CQ | Campherchinon |
| EHA | Speedcure EHA; 2-Ethylhexyl-4-(dimethylamino)benzoat (CAS 21245-02-3) |
| BHT | 3,5-Di-tert-butyl-4-hydroxytoluol (CAS 128-37-0) |
| EDMAB | Ethyl (N,N-dimethylamino)benzoat (CAS: 10287-53-3) |
| DPIHFP | Diphenyliodonium-hexafluorophosphat (CAS: 58109-40-3) |
| PEGDMA 400 | Polyethylenglykoldimethacrylat (CAS: 25852-47-5) |

### Messung der Haftfestigkeit (SBS-Wert, Shear Bond Strength)

Hierzu wurden zunächst bovine Schneidezähne ohne Pulpen in ein kalt polymerisierendes Harz eingebettet (Viscovoss GTS mit MEKP MEH-Härter; Voss Chemie). Unmittelbar vor Gebrauch wurden die eingebetteten Zähne bis zum Schmelz nass abgeschliffen (Schleifpapier P120) und mit einem feinen Schleifpapier (P500) nass nachgeschliffen. Bis zur Verwendung wurden die Zähne in demineralisiertem Wasser gelagert.

Vor Beginn der Messung wurde mittels ölfreier Druckluft die Feuchtigkeit von der beschliffenen Oberfläche entfernt. Das Adhäsiv wurde 10 s mit einem Pinsel aufgetragen und einmassiert. Nach weiteren 20 s Einwirkzeit wurden die Lösungsmittel vorsichtig verblasen und dann die Oberfläche 10 s mit einer Dentallampe (MiniLED, ACTEON Germany, Mettmann, Deutschland) belichtet. Anschließend wurde eine zweiteilige Teflonform mit einer Bohrung von 3,0 mm Durchmesser nach ISO/TS 11405:2003 aufgesetzt und mit einer Metallklammer fixiert. Die Bohrung wurde mit einem dentalen Füllungsmaterial (Komposit) (Ecusit™, Dental-Material GmbH, Deutschland) gefüllt und 40 s belichtet (MiniLED). Nach Aushärtung wurde die Teflonform entfernt und überstehende Reste des ausgehärteten Adhäsivs mit einem Skalpell entfernt.

Die so präparierten Prüfkörper wurden 23 h bei 37°C und dann 1h bei 23°C gelagert. Die Prüfkörper wurden anschließend in eine Halterung zur Messung der Haftfestigkeit (SBS-Wert) eingespannt (Abschervorrichtung gemäß ISO10477:2004) und in einer Apparatur zur Bestimmung eines Kraft-Weg-Diagramms (Z010/TN2A, Zwick GmbH & Co, Deutschland) bei einem Vorschub von 0,5 mm/min vermessen.

Die Prüfung wurde jeweils an 10 Prüfkörpern durchgeführt. Die SBS-Werte (angegeben ist der Mittelwert mit Standardabweichung) sind in Tabelle 3 wiedergegeben.

### Referenzzusammensetzung

Um die gemessenen Haftwerte mit Haftwerten des Standes der Technik vergleichen zu können wurde die Haftfestigkeit einer Referenzzusammensetzung mit der hier verwendeten Abschervorrichtung und zusätzlich mit der hier verwendeten sonstigen Messme-thode ermittelt.

Verwendet wurde ein Adhäsiv nach Beispiel 12 der EP 2 407 142 A1, enthaltend 6-(N-Methacrylamido)-1-hydroxy-hexanbisphosphonsäure **(MHbisP).** Die Zusammensetzung ist noch einmal in Tabelle 2 wiedergegeben. Für dieses Adhäsiv ist in der zitierten Schrift ein SBS-Wert auf Schmelz von 23,4 ± 6,7 MPa angegeben.

Das Beispiel 12 der EP 2 407 142 A1 wurde unter Verwendung der oben beschriebenen Teflonform und Abschervorrichtung exakt nachgearbeitet. Als Füllungsmaterial (Komposit) wurde Filtek™ Z250, (3M Espe, Deutschland) verwendet. Der ermittelte SBS-Wert betrug 11,9 ± 5,0 MPa.

Die oben beschriebene Messmethode ergab einem SBS-Wert von 9,7 ± 3,4 MPa. Eine Verbesserung der Haftkraft vorliegender Beispielzusammensetzungen gegenüber der Referenzzusammensetzung ergibt sich also bei Werten oberhalb 9,7 ± 3,4 MPa oder 11,9 ± 5,0 MPa.

**Tabelle 2: Referenzzusammensetzung (EP2407142A1)**

| | Gewichts-[%] |
|---|---|
| MHbisP | 7,5 |
| PEGDMA 400 | 5 |
| HEMA | 45,5 |
| CQ | 0, 8 |
| EDMAB | 0, 65 |
| DPIHFP | 0, 55 |
| Wasser | 40 |

### Beispieladhäsive

**Tabelle 3: Zusammensetzung der Beispieladhäsive**

| | **Vergleichsbeispiel 1** | **Vergleichsbeispiel 2** | **Vergleichsbeispiel 3** | **Erfindungsgemäßes Beispiel** |
|---|---|---|---|---|
| | **[Gew. -%]** | **[Gew. -%]** | **[Gew. -%]** | **[Gew. -%]** |
| AUbisPS | 10,6 | | | |
| PAUbisPS | | | | 10,6 |
| PAHbisPS | | 10,6 | | |
| AUbisP | | | 1 | |
| BisGMA | 24, 8 | 24, 8 | 24,8 | 24, 8 |
| HEMA | 29, 6 | 29, 6 | 29, 6 | 29,6 |
| Ethanol | 19, 8 | 19, 8 | 29, 4 | 19,8 |
| dest. Wasser | 13 | 13 | 13 | 13 |
| CQ | 0, 8 | 0, 8 | 0,8 | 0,8 |
| EHA | 1,4 | 1,4 | 1, 4 | 1,4 |
| BHT | 0, 01 | 0, 01 | 0, 01 | 0,01 |
| SBS | 15,1 ± 2,7 | 13,2 ± 4,7 | 15,5 ± 7,3 | 21,7 ± 6,0 |

Das erfindungsgemäße Beispiel unterscheidet sich vom Vergleichsbeispiel 1 ausschließlich durch die Substitution des Amid-Wasserstoffs durch eine Propylgruppe.

Das erfindungsgemäße Beispiel unterscheidet sich vom Vergleichsbeispiel 2 ausschließlich durch die Kettenlänge des Spacers zwischen der Bisphosphongruppe und der Propyl-Acrylamidgruppe.

Diese Beispiele zeigen, dass die Alkylierung einer Acrylamidgruppe und eine Kettenlänge des Spacers zwischen der Bisphosphongruppe und der Acrylamidgruppe > 5 C-Atome zu einer Erhöhung der Haftkraft führt.

## Patentansprüche

1. Dentalmaterial, das enthält:
a. radikalisch polymerisierbare Monomere gemäß Formel I: 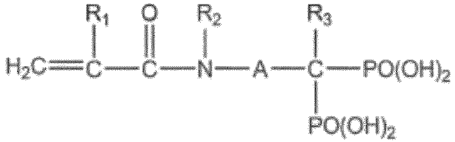 mit:
R1 = Methyl oder H
R2 = Alkyl, Aryl, oder Arylalkyl
R3 = H, Alkyl oder OR4
R4 = H oder Alkyl
A = C6-C18-Alkylen oder C8-C18-Alkylarylen;
b. radikalisch polymerisierbare Monomere enthaltend mindestens eine Acrylat- und/oder Methacrylatgruppe;
c. wenigstens einen Initiator für die radikalische Polymerisation.

2. Dentalmaterial nach Anspruch 1, **dadurch gekennzeichnet, dass** der Anteil radikalisch polymerisierbarer Monomere 10-99,98 Gew.-% beträgt.

3. Dentalmaterial nach Anspruch 1 oder 2, **dadurch gekennzeichnet, dass** der Anteil radikalisch polymerisierbarer Monomere der Merkmalsgruppe a. an der Gesamtmenge radikalisch polymerisierbarer Monomere 1-50 Gewichtsteile, bevorzugt 5 bis 50 Gewichtsteile beträgt; und dass der Anteil radikalisch polymerisierbarer Monomere der Merkmalsgruppe b. an der Gesamtmenge radikalisch polymerisierbarer Monomere 99-50 Gewichtsteile, bevorzugt 95 bis 50 Gewichtsteile beträgt.

4. Dentalmaterial nach einem der Ansprüche 1 bis 3, **dadurch gekennzeichnet, dass** ein oder mehrere, bevorzugt alle Substituenten der Formel 1 ausgewählt sind aus der Gruppe bestehend aus:
R1 = H
R2 = C1-C6-Alkyl
R3 = H
A = C8-C14-Alkylen.

5. Dentalmaterial nach einem der Ansprüche 1 bis 4, **dadurch gekennzeichnet, dass** der Anteil des wenigstens einen Initiators für die radikalische Polymerisation an der Gesamtmasse des Dentalmaterials 0,01-10 Gew.-% beträgt.

6. Dentalmaterial nach einem der Ansprüche 1 bis 5, **dadurch gekennzeichnet, dass** der wenigstens eine Initiator für die radikalische Polymerisation ein Photoinitiator ist, bevorzugt ein Photoinitiator für Wellenlängenbereiche zwischen 390 und 500 nm; und/oder dass der wenigstens eine Initiator für die radikalische Polymerisation ein aktiviertes Initiatorsystem ist.

7. Dentalmaterial nach einem der Ansprüche 1 bis 6, **dadurch gekennzeichnet, dass** es ein Initiatorsystem bestehend aus mindestens einem aktivierten Initiatorsystem und mindestens einem Fotoinitiatorsystem enthält.

8. Dentalmaterial nach einem der Ansprüche 1 bis 7, **dadurch gekennzeichnet, dass** es 0-90 Gew.-% mindestens eines Lösungsmittels ausgewählt aus der Gruppe bestehend aus Wasser und organisches Lösungsmittel enthält; wobei bevorzugte Untergrenzen für den Wasseranteil 0,1; 1 oder 5 Gew.-% und bevorzugte Obergrenzen für den Wasseranteil 20 oder 15 Gew.-% sind; und wobei bevorzugte Untergrenzen für den Anteil organisches Lösungsmittel 0,0; 0,1; 1 oder 5 Gew.-% und bevorzugte Obergrenzen für den Anteil organisches Lösungsmittel 90, 50 oder 20 Gew.-% sind.

9. Dentalmaterial nach Anspruch 8, **dadurch gekennzeichnet, dass** das organische Lösungsmittel ausgewählt ist aus der Gruppe bestehend aus bei Raumtemperatur (23 °C) flüchtigen Lösungsmitteln mit einer nach DIN 153170 bestimmten Verdunstungszahl < 80; bevorzugt aus der Gruppe bestehend aus Ethanol, Propanol und Butanol.

10. Dentalmaterial nach einem der Ansprüche 1 bis 9, **dadurch gekennzeichnet, dass** es 0,01-10 Gew.-% dentalüblicher Additive enthält, bevorzugt Additive ausgewählt aus der Gruppe bestehend aus Inhibitoren, Stabilisatoren, Beschleunigern, Farbmitteln, Fluoridierungsmitteln, Remineralisierungsmitteln, Röntgenopakern und zusätzlichen Filmbildnern.

11. Dentalmaterial nach einem der Ansprüche 1 bis 10, **dadurch gekennzeichnet, dass** es Füllstoffe enthält, wobei bevorzugt die Untergrenze des Anteils der Füllstoffe an der Gesamtmasse des Dentalmaterials 0,0; 0,1 oder 1 Gew.-% und wobei bevorzugt die Obergrenze des Anteils der Füllstoffe an der Gesamtmasse des Dentalmaterials 90, 80, 65 oder 45 Gew.-% beträgt; und wobei bevorzugt die Füllstoffe ausgewählt sind aus der Gruppe bestehend aus:
- Dentalgläsern, bevorzugt Dentalgläser mit einer mittleren Partikelgröße zwischen 200 nm und 50 µm, die weiterhin bevorzugt Acrylat- und/oder Methacrylatgruppen an ihrer Oberfläche aufweisen;
- pyrogenen Kieselsäuren;
- nass gefällten Kieselsäuren;
- Nanofüllstoffen mit einer mittleren Partikelgröße < 100 nm, die bevorzugt nicht aggregiert sind;
- Nanofüllstoffe mit einer mittleren Partikelgröße < 100 nm, die bevorzugt nicht aggregiert und nicht agglomeriert sind.

12. Dentalmaterial nach einem der Ansprüche 1 bis 11, **dadurch gekennzeichnet, dass** die radikalisch polymerisierbaren Monomere der Merkmalsgruppe b. keine Acrylamid- und/oder Methacrylamidgruppen aufweisen; bevorzugt keine radikalisch polymerisierbare Gruppe aufweisen, die verschieden ist von Acrylat- und/oder Methacrylatgruppen.

13. Dentalmaterial nach einem der Ansprüche 1 bis 12, **dadurch gekennzeichnet, dass** die radikalisch polymerisierbaren Monomere der Merkmalsgruppe b. keine Säuregruppen und/oder keine Polyoxyalkylengruppen mit mehr als vier Oxyalkyleneinheiten aufweisen, sofern diese mindestens zwei (Meth)acrylatgruppen enthalten.

14. Dentalmaterial nach einem der Ansprüche 1 bis 13, **dadurch gekennzeichnet, dass** es formuliert ist als ein Material ausgewählt aus der Gruppe bestehend aus Bondings, Adhäsiven, Fissurenversieglern, Befestigungszementen, Füllungsmaterialien und Kompositen.

15. Dentalmaterial nach einem der Ansprüche 1 bis 13 zur Verwendung als Bonding, Adhäsiv, Fissurenversiegler, Befestigungszement, Füllungsmaterial oder Komposit.

## Claims

1. Dental material, comprising:
a. radically polymerizable monomers according to formula I: 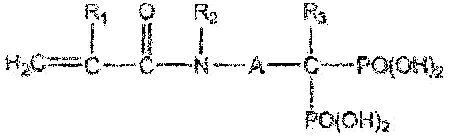 wherein:
R₁ = methyl or H
R₂ = alkyl, aryl or arylalkyl
R₃ = H, alkyl or OR₄
R₄ = H or alkyl
A = C₆-C₁₈ alkylene or C₈-C₁₈ alkylarylene;
b. radically polymerizable monomers comprising at least one acrylate and/or methacrylate group;
c. at least one initiator for the radical polymerization.

2. Dental material according to Claim 1, **characterized in that** the proportion of radically polymerizable monomers is 10-99.98% by weight.

3. Dental material according to Claim 1 or 2, **characterized in that** the proportion of radically polymerizable monomers of the feature group a. in the total amount of radically polymerizable monomers is 1 to 50 parts by weight, preferably 5 to 50 parts by weight; and that the proportion of radically polymerizable monomers of the feature group b. in the total amount of radically polymerizable monomers is 99 to 50 parts by weight, preferably 95 to 50 parts by weight.

4. Dental material according to any of Claims 1 to 3, **characterized in that** one or more, preferably all, substituents of the formula 1 are selected from the group consisting of:
R₁ = H
R₂ = C₁-C₆ alkyl
R₃ = H
A = C₈-C₁₄ alkylene.

5. Dental material according to any of Claims 1 to 4, **characterized in that** the proportion of the at least one initiator for the radical polymerization in the total mass of the dental material is 0.01-10% by weight.

6. Dental material according to any of Claims 1 to 5, **characterized in that** the at least one initiator for the radical polymerization is a photoinitiator, preferably a photoinitiator for wavelength regions between 390 and 500 nm; and/or that the at least one initiator for the radical polymerization is an activated initiator system.

7. Dental material according to any of Claims 1 to 6, **characterized in that** it comprises an initiator system consisting of at least one activated initiator system and at least one photoinitiator system.

8. Dental material according to any of Claims 1 to 7, **characterized in that** it comprises 0-90% by weight of at least one solvent selected from the group consisting of water and organic solvents; wherein preferred lower limits for the water proportion are 0.1, 1 or 5% by weight and preferred upper limits for the water proportion are 20 or 15% by weight; and wherein preferred lower limits for the proportion of organic solvents are 0.0, 0.1, 1 or 5% by weight and preferred upper limits for the proportion of organic solvents are 90, 50 or 20% by weight.

9. Dental material according to Claim 8, **characterized in that** the organic solvent is selected from the group consisting of at room temperature (23°C) volatile solvents with an evaporation number determined in accordance with DIN 153170 of < 80; preferably from the group consisting of ethanol, propanol and butanol.

10. Dental material according to any of Claims 1 to 9, **characterized in that** it comprises 0.01-10% by weight of customary dental additives, preferably additives selected from the group consisting of inhibitors, stabilizers, accelerators, colorants, fluoridation agents, remineralization agents, X-ray opacifiers and additional film-formers.

11. Dental material according to any of Claims 1 to 10, **characterized in that** it comprises fillers, wherein the lower limit of the proportion of the fillers in the total mass of the dental material is preferably 0.0, 0.1 or 1% by weight and wherein the upper limit of the proportion of fillers in the total mass of the dental material is preferably 90, 80, 65 or 45% by weight; and wherein the fillers are preferably selected from the group consisting of:
- dental glasses, preferably dental glasses having an average particle size between 200 nm and 50 µm, which more preferably have acrylate and/or methacrylate groups on their surface;
- fumed silicas;
- wet-precipitated silicas;
- nanofillers having an average particle size of < 100 nm that are preferably not aggregated;
- nanofillers having an average particle size of < 100 nm that are preferably not aggregated and not agglomerated.

12. Dental material according to any of Claims 1 to 11, **characterized in that** the radically polymerizable monomers of the feature group b. have no acrylamide and/or methacrylamide groups; preferably have no radically polymerizable group, which is different from acrylate and/or methacrylate groups.

13. Dental material according to any of Claims 1 to 12, **characterized in that** the radically polymerizable monomers of the feature group b. have no acid groups and/or no polyoxyalkylene groups having more than four oxyalkylene units, where these contain at least two (meth)acrylate groups.

14. Dental material according to any of Claims 1 to 13, **characterized in that** it is formulated as a material selected from the group consisting of bondings, adhesives, fissure sealants, fixing cements, filling materials and composites.

15. Dental material according to any of Claims 1 to 13 for use as a bonding, adhesive, fissure sealant, fixing cement, filling material or composite.

## Revendications

1. Matériau dentaire, qui contient :
a. des monomères polymérisables de manière radicalaire selon la formule I : 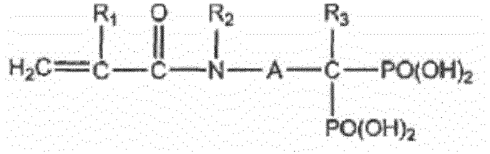 avec :
R₁ = méthyle ou H
R₂ = alkyle, aryle, ou arylalkyle
R₃ = H, alkyle ou OR₄
R₄ = H ou alkyle
A = C6-C18-alkylène ou C8-C18-alkylarylène ;
b. des monomères polymérisables de manière radicalaire contenant au moins un groupe acrylate et/ou méthacrylate ;
c. au moins un initiateur pour la polymérisation de manière radicalaire.

2. Matériau dentaire selon la revendication 1, **caractérisé en ce que** la proportion de monomères polymérisables de manière radicalaire est de 10 à 99,98 % en poids.

3. Matériau dentaire selon la revendication 1 ou 2, **caractérisé en ce que** la proportion de monomères polymérisables de manière radicalaire du groupe de caractéristiques a. par rapport à la quantité totale de monomères polymérisables de manière radicalaire est de 1 à 50 parties en poids, préférablement 5 à 50 parties en poids ; et **en ce que** la proportion de monomères polymérisables de manière radicalaire du groupe de caractéristiques b. par rapport à la quantité totale de monomères polymérisables de manière radicalaire est de 99 à 50 parties en poids, préférablement 95 à 50 parties en poids.

4. Matériau dentaire selon l'une quelconque des revendications 1 à 3, **caractérisé en ce qu'**un ou plusieurs substituants, préférablement tous les substituants de la formule 1 sont choisis dans le groupe constitué par :
R₁ = H
R₂ = C1-C6-alkyle
R₃ = H
A = C8-C14-alkylène.

5. Matériau dentaire selon l'une quelconque des revendications 1 à 4, **caractérisé en ce que** la proportion de l'au moins un initiateur pour la polymérisation de manière radicalaire par rapport à la masse totale du matériau dentaire est de 0,01 à 10 % en poids.

6. Matériau dentaire selon l'une quelconque des revendications 1 à 5, **caractérisé en ce que** l'au moins un initiateur pour la polymérisation de manière radicalaire est un photoinitiateur, préférablement un photoinitiateur pour des domaines de longueur d'onde entre 390 et 500 nm ; et/ou **en ce que** l'au moins un initiateur pour la polymérisation de manière radicalaire est un système d'initiateur activé.

7. Matériau dentaire selon l'une quelconque des revendications 1 à 6, **caractérisé en ce qu'**il contient un système d'initiateur constitué d'au moins un système d'initiateur activé et d'au moins un système de photoinitiateur.

8. Matériau dentaire selon l'une quelconque des revendications 1 à 7, **caractérisé en ce qu'**il contient 0 à 90 % en poids d'au moins un solvant choisi dans le groupe constitué par l'eau et un solvant organique ; les limites inférieures préférées pour la proportion en eau étant de 0,1 ; 1 ou 5 % en poids et les limites supérieures préférées pour la proportion en eau étant de 20 ou 15 % en poids ; et les limites inférieures préférées pour la proportion en solvant organique étant de 0,0 ; 0,1 ; 1 ou 5 % en poids et les limites supérieures préférées pour la proportion en solvant organique étant de 90, 50 ou 20 % en poids.

9. Matériau dentaire selon la revendication 8, **caractérisé en ce que** le solvant organique est choisi dans le groupe constitué par des solvants volatils à température ambiante (23 °C) dotés d'un indice d'évaporation < 80, déterminé d'après la norme DIN 153170 ; préférablement choisi dans le groupe constitué par l'éthanol, le propanol et le butanol.

10. Matériau dentaire selon l'une quelconque des revendications 1 à 9, **caractérisé en ce qu'**il contient 0,01 à 10 % en poids d'additifs habituels dans le domaine dentaire, préférablement des additifs choisis dans le groupe constitué par des inhibiteurs, des stabilisants, des accélérateurs, des colorants, des agents de fluoration, des agents de reminéralisation, des agents opacifiants aux rayons X et des agents filmogènes supplémentaires.

11. Matériau dentaire selon l'une quelconque des revendications 1 à 10, **caractérisé en ce qu'**il contient des charges, préférablement la limite inférieure de la proportion des charges dans la masse totale du matériau dentaire étant de 0,0 ; 0,1 ou 1 % en poids et préférablement la limite supérieure de la proportion des charges dans la masse totale du matériau dentaire étant de 90, 80, 65 ou 45 % en poids ; et préférablement les charges étant choisies dans le groupe constitué par :
- des verres dentaires, préférablement des verres dentaires dotés d'une taille moyenne de particule comprise entre 200 nm et 50 µm, qui présentent en outre préférablement des groupes acrylate et/ou méthacrylate au niveau de leur surface ;
- des silices pyrogénées ;
- des silices précipitées par voie humide ;
- des nanocharges dotées d'une taille moyenne de particule < 100 nm, qui ne sont préférablement pas agrégées ;
- des nanocharges dotées d'une taille moyenne de particule < 100 nm, qui ne sont préférablement pas agrégées et pas agglomérées.

12. Matériau dentaire selon l'une quelconque des revendications 1 à 11, **caractérisé en ce que** les monomères polymérisables de manière radicalaire du groupe de caractéristiques b. ne présentent aucun groupe acrylamide et/ou méthacrylamide ; préférablement ne présentent aucun groupe polymérisable de manière radicalaire qui est différent de groupes acrylate et/ou méthacrylate.

13. Matériau dentaire selon l'une quelconque des revendications 1 à 12, **caractérisé en ce que** les monomères polymérisables de manière radicalaire du groupe de caractéristiques b. ne présentent aucun groupe de type acide et/ou aucun groupe polyoxyalkylène comportant plus de quatre motifs oxyalkylène, pour autant que ceux-ci contiennent au moins deux groupes (méth)acrylate.

14. Matériau dentaire selon l'une quelconque des revendications 1 à 13, **caractérisé en ce qu'**il est formulé en tant que matériau choisi dans le groupe constitué par des bondings, des adhésifs, des agents de scellement de fissure, des ciments de fixation, des matériaux de remplissage et des composites.

15. Matériau dentaire selon l'une quelconque des revendications 1 à 13 pour une utilisation en tant que bonding, adhésif, agent de scellement de fissure, ciment de fixation, matériau de remplissage ou composite.
